(19) Europäisches Patentamt
European Patent Office
Office européen des brevets

(11) Publication number: **0 107 306**
**B1**

(12) # EUROPEAN PATENT SPECIFICATION

(45) Date of publication of patent specification: **30.04.86**

(51) Int. Cl.⁴: **C 07 D 233/50,** A 61 K 31/415

(21) Application number: **83305194.9**

(22) Date of filing: **07.09.83**

(54) **Imidazoline derivatives.**

(30) Priority: **16.09.82 GB 8226455**

(43) Date of publication of application:
**02.05.84 Bulletin 84/18**

(45) Publication of the grant of the patent:
**30.04.86 Bulletin 86/18**

(84) Designated Contracting States:
**BE CH DE FR GB IT LI NL SE**

(56) References cited:
**EP-A-0 035 393**
**EP-A-0 043 659**
**EP-A-0 070 084**

(73) Proprietor: **BEECHAM GROUP PLC**
**Beecham House Great West Road**
**Brentford Middlesex TW8 9BD (GB)**

(72) Inventor: **Newsome, Peter Martin**
**The Limes 20A York Road**
**Cheam Surrey (GB)**
Inventor: **Moss, Stephen Frederick**
**40 Blakehall Road**
**Carshalton Surrey (GB)**
Inventor: **Beeley, Lee James**
**40 St. Johns Road**
**Westcott Dorking Surrey (GB)**
Inventor: **Burgess, Malcolm Neil**
**17 Englefield Hills Farm Lane**
**Horsham Sussex (GB)**

(74) Representative: **Cresswell, Thomas Anthony et al**
**European Patent Attorney Beecham Pharmaceuticals Great Burgh Yew Tree Bottom Road**
**Epsom Surrey KT18 5XQ (GB)**

**EP 0 107 306 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to quaternary aminophenyliminoimidazolidines and to their use in treating diarrhoea in humans and scours in animals, particularly to treatment of enterotoxin induced diarrhoea.

In EP—A—43 659 there are disclosed imidazoline derivatives having anti-diarrhoeal activity.

According to the present invention there is provided a compound of formula (I):

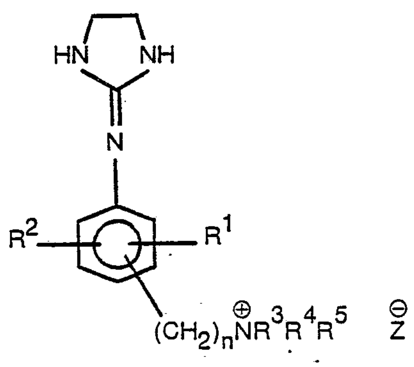

(I)

wherein $R^1$ and $R^2$ are the same or different and each is selected from halogen, alkyl, haloalkyl, alkoxy and alkoxyalkyl,

$R^3$, $R^4$ and $R^5$ are the same or different and each is alkyl;

wherein the alkyl and alkoxy groups each have from 1 to 6 carbon atoms; those having 3 or more carbon atoms may be straight or branched chains and wherein the haloalkyl groups have 1, 2 or 3 halogen atoms;

n is 0, 1, 2 or 3; and

Z is a pharmaceutically or veterinarily acceptable anion,

and salts thereof.

Particular examples of haloalkyl groups are the trifluoromethyl and 2,2,2-trichloroethyl groups.

Preferably $R^1$ and $R^2$ are the same and each is methyl or chloro.

Preferably $R^1$ and $R^2$ are located in the 2,6 positions in relation to the imidazolidinimino group.

Suitable pharmaceutically or veterinarily acceptable anions include halide, hydroxide, sulphate and hydrogen sulphate anions, or carboxylate such as acetate and citrate.

Suitable salts include pharmaceutically or veterinarily acceptable salts, but it is not essential that salts are pharmaceutically or veterinarily acceptable as such salts may also be useful in producing or purifying the desired compound of formula (I). Pharmaceutically and veterinarily acceptable salts include acid addition salts with pharmaceutically or veterinarily acceptable salts, including hydrochloric, hydrobromic, hydroiodic, nitric, sulphuric, citric, lactic, maleic, pamoic and tartaric acids.

The compounds of formula (I) have advantages in the treatment of diarrhoea and scours, especially hypersecretory diarrhoeas and protect animals from death due to enteropathogenic *E. coli* infection of the gastro-intestinal tract.

Accordingly, the present invention provides a compound of formula (I) for use in human or veterinary medicine, especially for use in treating or preventing diarrhoea in humans and scours in animals.

The present invention also provides a process for producing a compound of formula (I), which process comprises either

(a) reacting a compound of formula (II)

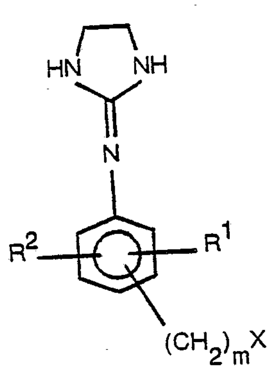

(II)

or a salt thereof,

wherein

$R^1$, and $R^2$ are as defined in relation to formula (I) and m is 1, 2 or 3 and

X is a leaving group, preferably halogen with a compound of formula (III)

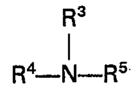

(III)

wherein

$R^3$, $R^4$ and $R^5$ are as defined in relation to formula (I) or

(b) reacting a compound of formula (IV)

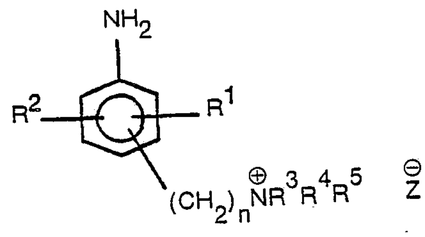

(IV)

wherein

$R^1$, $R^2$, $R^3$, $R^4$, $R^5$, n and $Z^{\ominus}$ are as defined in relation to formula (I)

with a compound of formula (V)

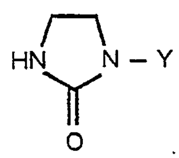

(V)

wherein Y is a protecting group and thereafter removing the protecting group, or

(c) reacting a compound of formula (VI):

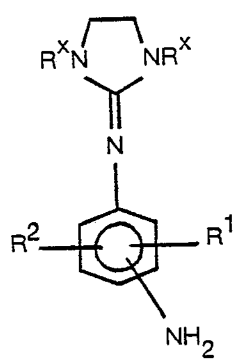

(VI)

wherein

$R^1$ and $R^2$ are as defined in relation to formula (I) and $R^x$ is a suitable protecting group with one or more alkyl halides

and thereafter removing the protecting groups $R^x$.

The reaction of a compound or formula (II) with a compound of formula (III) is suitably conducted in an organic solvent, such as a lower alkanol, at ambient temperature. Advantageously a salt of the compound of formula (II), especially a hydrohalide salt is employed and the leaving group X forms the anion $Z^{\ominus}$ during the reaction.

The reaction of a comound of formula (IV) with a compound of formula (V) is suitably conducted in the presence of phosphoryl chloride at elevated temperature. The protecting group Y may be a conventional protecting group, such as an acyl group, for instance an acetyl group, and may be removed by conventional methods, such as by treating with an acid or base.

The reaction of a compound of formula (VI) with an alkyl halide may be effected under conventional conditions. Preferably an alkyl bromide is used. When $R^3$, $R^4$ and $R^5$ are different, appropriate alkyl halides may be used. The protecting groups $R^x$ are suitable conventional protecting groups, such as trialkylsilyl groups, especially trimethylsilyl groups, which may be removed under conventional conditions.

Compounds of Formula (II), (IV) and (VI) are readily produced from known compounds, for instance using the reactions shown in Schemes I to IV:

SCHEME I

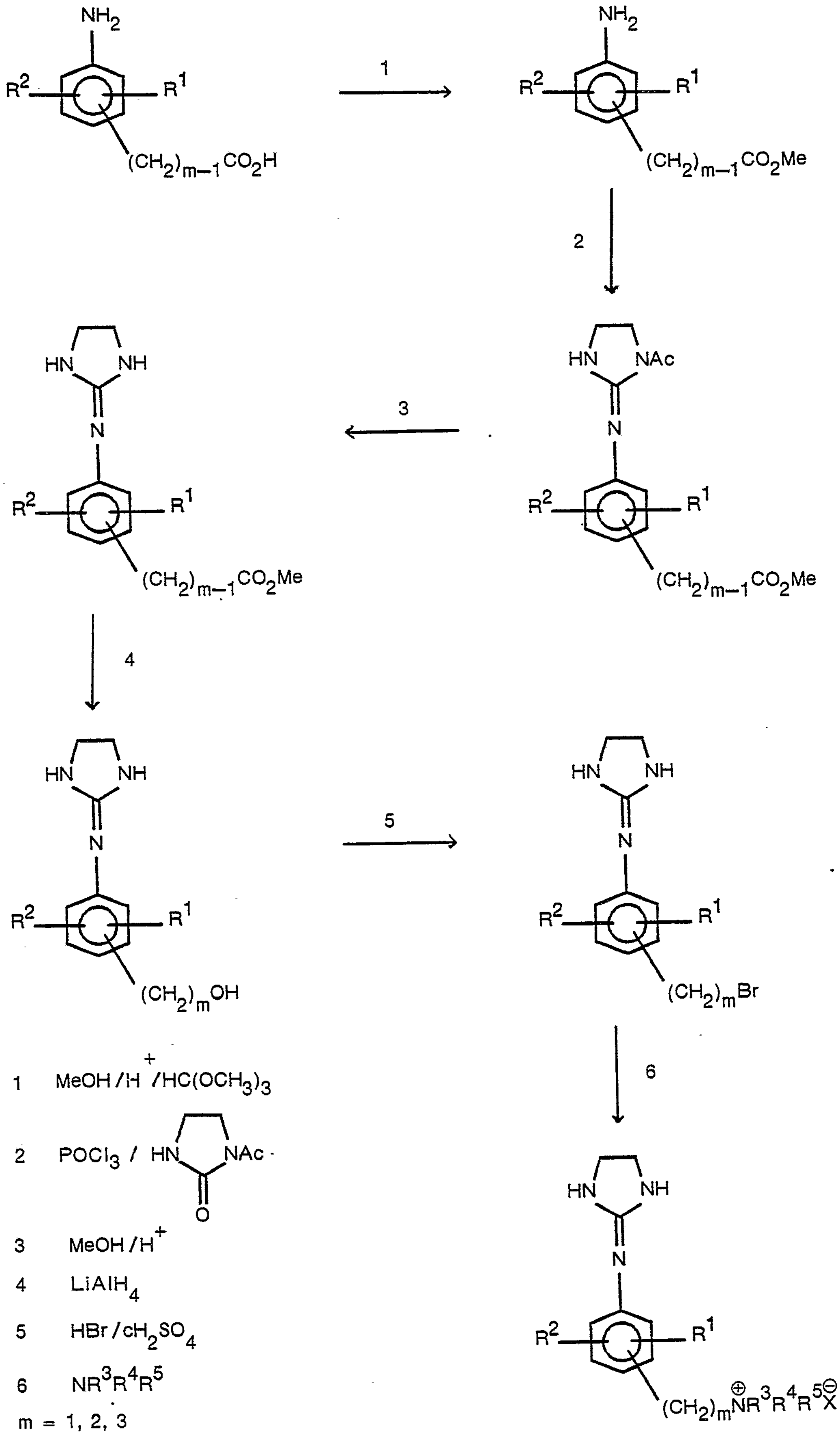
1
2
3
4
5
6
1 MeOH / H+ / HC(OCH3)3
2 POCl3 /
3 MeOH / H+
4 LiAlH4
5 HBr / cH2SO4
6 NR3R4R5
m = 1, 2, 3

SCHEME II

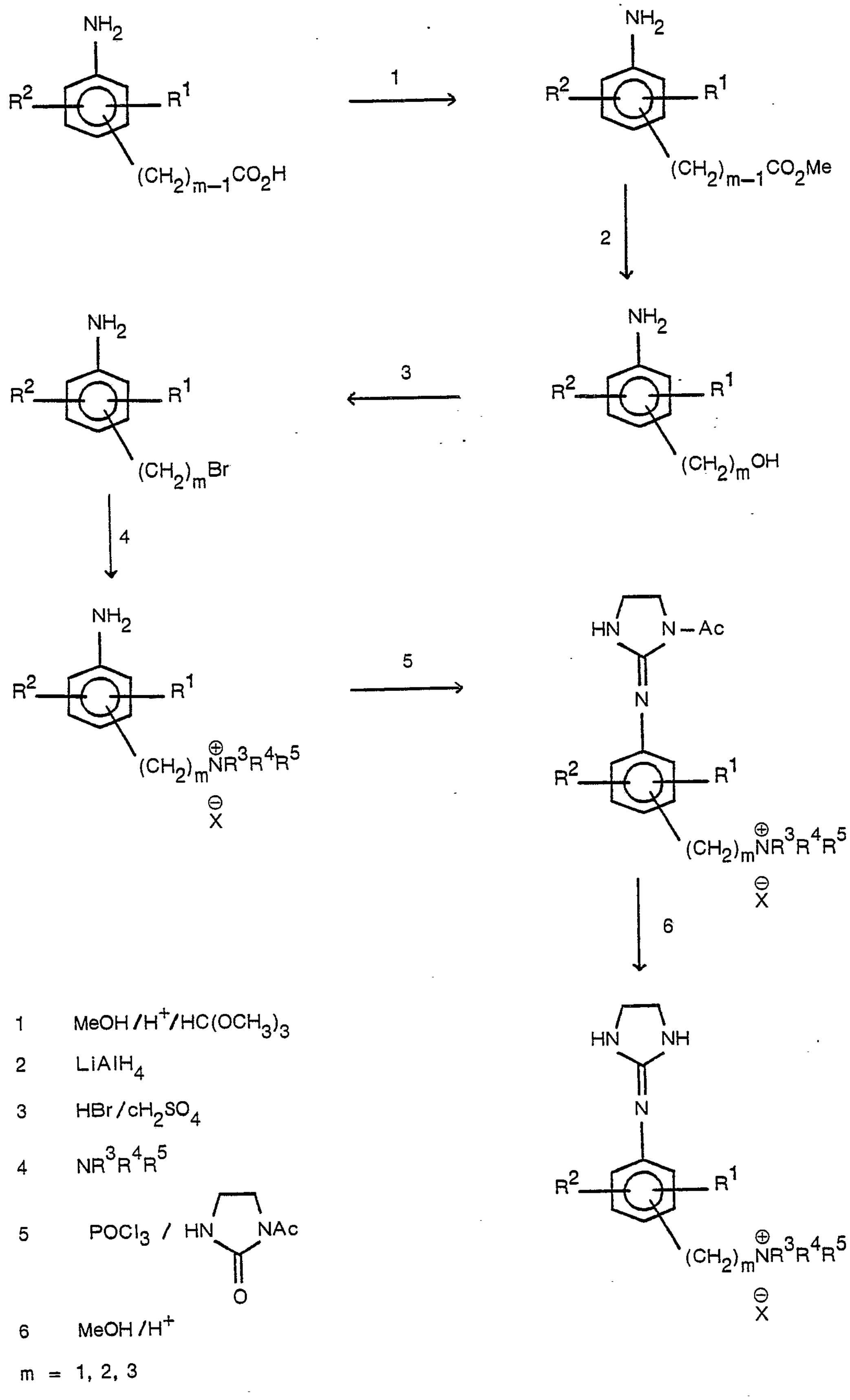
1
2
3
4
5
6
1 $MeOH / H^+ / HC(OCH_3)_3$
2 $LiAlH_4$
3 $HBr / cH_2SO_4$
4 $NR^3R^4R^5$
5 $POCl_3$ /
6 $MeOH / H^+$
m = 1, 2, 3

SCHEME III

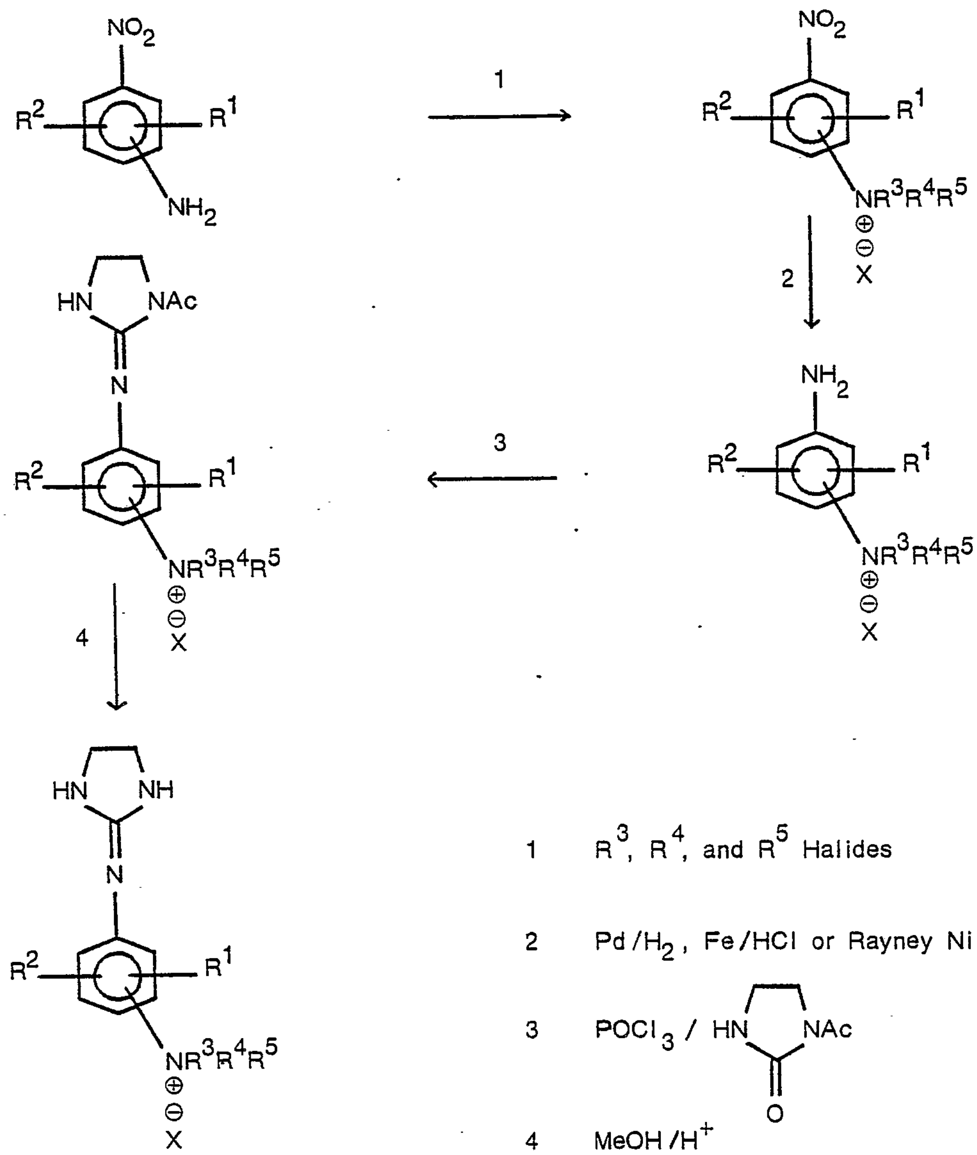
1
2
3
4
1 R3, R4, and R5 Halides
2 Pd/H2, Fe/HCl or Rayney Ni
3 POCl3 /
4 MeOH/H+

SCHEME IV

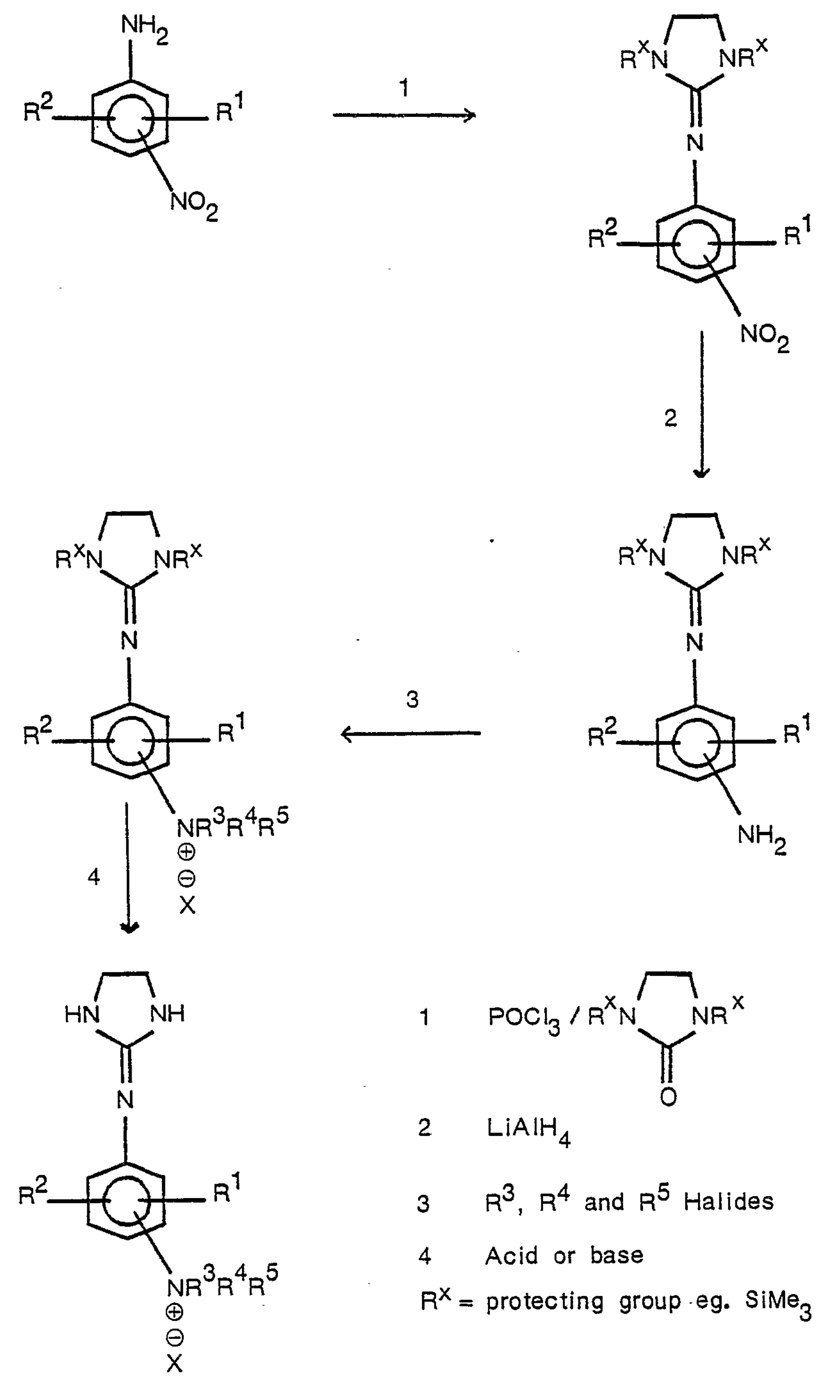


The present invention further provides a pharmaceutical or veterinary composition comprising a compound of formula (I) (hereinafter referred to as the "drug") and a pharmaceutically or veterinarily acceptable carrier therefor.

Pharmaceutical and veterinary compositions of the drug will, of course, be adapted for administration to the humans or animals to be treated. Thus, for example, the composition may be a shaped composition, such as bolus, tablet or capsule. In such cases the pharmaceutically or veterinarily acceptable carrier will be chosen from the usual range of lubricants, dispersants, binders, fillers and the like. When these shaped compositions are for administration to cattle and pigs often they may for instance weigh at least 1 g, on occasions at least 2 g.

For administration to humans, especially children, the drug may suitably be presented as a syrup including suitable colouring and/or flavouring agents. Such syrups are conveniently presented in unit or multi-dose containers.

For veterinary use the composition may also be a dispersion or a solution of the drug in a suitable

vehicle for use with an oral doser (this is a well known item of farm equipment, basically comprising a liquid reservoir, a mouthpiece adapted for insertion into animal mouths, and a pump mechanism whereby unit doses can be ejected from the reservoir through the mouthpiece). Conveniently the drug may be administered from an oral doser as an aqueous solution. Alternatively, the vehicle will be an oil or water based cream to ensure homogeneity of the unit does administered.

The invention, therefore, also provides an oral doser containing a multi-dose of the drug in a veterinarily acceptable vehicle.

The drugs of the invention may also be added to the animal feed or drinking water. Thus the invention also provides animal feed or animal drinking water containing a compound of formula (I). It will be convenient to formulate these animal feed and drinking water compositions with a multi-dose of the drug so that the animal takes in an appropriate quantity of the drug along with its diet. It will also be convenient to present the composition of the invention as a pre-mix for addition to the feed or drinking water.

With human babies or young animals, a particularly useful technique is to blend their milk with the drugs of this invention.

The compositions of the invention may also be formulated for injection. In such cases the drug chosen is suitably dissolved in water for injection. Alternatively the drug may be administered in a solution used for parenteral fluid replacement therapy.

Often it will be appropriate to include in the compositions a further medicine such as an antibacterial agent for example an antibiotic such as amoxycillin or neomycin or a sulphonamide such as sulfadoxin, an agent to alter intestinal motility such as loperamide or a material such as pectin.

Treatment of diarrhoea and scours using the drug may be supplemented by oral rehydration therapy such as those described in U.K. Patent No. 1,581,826 and German Offenlegungsschrift No. 28 54 281, UK Patent Application No. 2 012 163A, US Patent No. 3 898 328, Nalin, D.R. and Cash, R.A., Bull. World Health Org., *43*, 361 (1970), French Patent No. 2 467 599, UK Patent No. 1 465 308 and as described in "Secretory Diarrhoea", Ed M. Field, J. S. Fordtran and S. G. Schultz, American Physiological Society, Maryland, 1980 pp 179—185 and Lancet, (1975) pp 79 and 80. Conveniently the drug may be administered with the oral rehydration formulation.

Accordingly the present invention provides, in a particular aspect, a formulation for treating diarrhoea which comprises an effective non-toxic amount of a compound of formula (I) as hereinbefore defined and an oral rehydration composition comprising a pharmacologically acceptable aqueous solution containing at least 0.5% w/v of an actively absorbed monosaccharide, at least 25 mM sodium ions and having an osmolarity less than 500 m Osmolar.

Preferably the oral rehydration composition further comprises actively-absorbed amino acids and electrolytes.

The drug may be presented as a formulation containing or or more components of the oral rehydration composition for admixture with the remaining components.

Alternatively the drug may be provided separately and administered simultaneously or sequentially with the oral rehydration formulation.

The amount of drug administered must, of course, be sufficient to bring about the desired effect and will also depend on the body weight of the recipient and the chosen route of administraton. Typical dosages are in the range from 0.1 to 100 mg/kg particularly from 1 to 50 mg/kg. Useful dosage units based on such dosage would contain from 0.1 mg to 2500 mg of the drug, more suitably 1 mg to 2500 mg. Of course, it will be appreciated that many preferred compositions of the invention are in multi-dose form as, for the therapy of animals, it is often most desirable to be able rapidly to treat a number of animals. Such multi-dose compositions will contain, by way of example, at least 10 mg of the drug. Depending on the exact nature of the said multi-dose composition, often it will contain at least 250 mg of the drug, and on occasions as much as 25 g. Doses may be administered once or several times daily.

The present invention will now be illustrated by the following Examples which are not intended to limit the invention in any way.

Example 1

2-(2,6-Dichloro-4-triethylammoniomethylphenylimino)imidazolidine bromide

A solution of 2 - (4 - bromomethyl - 2,6 - dichlorophenylimino)imidazolidine hydrobromide (1.75 g, 4.3 mmol) in ethanol (60 $cm^3$) was added over 0.3 h to stirred triethylamine (8.75 $cm^3$) at room temperature. After 2.5h the reaction mixture was evaporated to a colourless oil which was dissolved in chloroform (30 $cm^3$) and the solution cooled and left for 24h. The suspended white solid was filtered, washed with chloroform and twice recrystallised from ethanol/diethyl ether affording prisms of 2 - (2,6 - dichloro - 4 - triethylammoniomethylphenylimino)imidazolidine bromide (1.04 g 2.5 mmol) m.p. > 260°C

*Analysis* calculated for $C_{16}H_{25}BrCl_2N_4$
Theory: C, 45.30; H, 5.93; Br, 18.81; Cl, 16.68; N, 13.19%
Found: C, 45.55; H, 5.85; Br, 18.74; Cl, 16.91; N, 13.11%

$^1H$ nmr (60 MHz) δH [$(CD_3)_2SO$]
7.44 (s, 2H, Ar-H), 6.24 (br, 2H, NH), 4.43 (S, 2H, $ArCH_2$), 3.20 (q, 6H, $CH_2CH_3$), 1.30 ppm (t, 9H, $CH_2CH_3$)

Example 2

2-(4-Bromomethyl-2,6-dichlorophenylimino)imidazolidine hydrobromide

Concentrated sulphuric acid (1.0 $cm^3$) was added dropwise to a stirred suspension of 2 - (2,6 - dichloro - 4 - hydroxymethylphenylimino)imidazolidine (2.34 g, 9.0 mmol) in 48% hydrobromic acid (4.7 $cm^3$). The suspension was heated to 100°C for 1.5h then cooled and diluted with acetone (20 $cm^3$). The suspended white prisms were filtered off and identified as 2 - (4 - bromomethyl - 2,6 - dichlorophenylimino)imidazolidine hydrobromide (2.79 g, 6.9 mmol) m.p. 265—6°C (dec.).

*Analysis* calculated for $C_{10}H_{11}Br_2Cl_2N_3$
Theory: C, 29.73; H, 2.74; N, 10.38%
Found: C, 30.03; H, 2.64; N, 10.25%

$^1H$ (60 Mz) δH [$(CD_3)_2SO$]

10.60 (br, 1H, $\overset{\oplus}{N}H$), 8.59 (br, 2H, NH), 7.80 (S, 2*H*, Ar-H), 4.80 (s, 2H, $CH_2Br$), 3.80 ppm (s, 4H, $(CH_2)_2$)

Example 3

2-(2,6-dichloro-4-hydroxymethylphenylimino)imidazolidine

Lithium aluminium hydride (300 mg, 7.9 mM) was added to a stirred suspension of 2 - (4 - carbomethoxy - 2,6 - dichlorophenylimino)imidazolidine (750 mg, 2.6 mM) in dry tetrahydrofuran (50 ml) with stirring. The mixture was then stirred at room temperature for several hours after which time ethyl acetate was carefully added. The solvent was then removed under vacuum and water was added to the residue. After basification with dilute sodium hydroxide the aqueous mixture was extracted several times with ethyl acetate. The extract (750 mg) was dried and evaporated to give a solid which was recrystallised from isopropanol to give a white solid (330 mg) m.p. 208—210°C.

*Microanalysis*

Calc. for $C_{10}H_{11}Cl_2N_3O$
C, 46.17; H, 4.26; N, 16.15%
Found C, 46.23; H, 4.12; N, 15.9%.

Example 4

2-(4-Carbomethoxy-2,6-dichlorophenylimino)imidazolidine

A solution of 1 - acetyl - 2 - (4 - carbomethoxy - 2,6 - dichloropenylimino)imidazolidine (5 g, 15 mmole) and 2M hydrochloric acid (1 ml, 2 mmole) in methanol (100 ml) was heated under reflux for 40 hours. The mixture was then evaporated to give a solid which was taken up in methylene chloride/methanol (650 ml:30 ml) and extracted with saturated sodium carbonate (300 ml). The organic layer was dried and evaporated to give a residue which after trituration with ether, yielded the title compound as a solid (3.5 g) mp 229—331°C.

*Analysis* calculated for $C_{11}H_{11}Cl_2N_3O_2$
Theory: C, 45.85; H, 3.85; N, 14.58
Found: C, 46.20; H, 3.83; N, 14.79

Example 5

1-Acetyl-2-(4-carbomethoxy-2,6-dichlorophenylimino)imidazolidine

Methyl-4-amino-3,5-dichlorobenzoate (16.8 g, 31 mmole), 1-acetyl-2-imidazolidone (4.3 g, 33.5 mmole) in phosporyl chloride (44 ml) were stirred at 50°C for 3 days. After cooling the phosphoryl chloride was evaporated to give an oily residue. Iced water was added to the residue which was then basified with aqueous sodium hydroxide. The mixture was extracted with methylene chloride which was then washed with water, dried (magnesium sulphate) and evaporated to give a creamy solid. This solid was recrystallised from toluene to give the title compound (7.7 g) mp 188—189°C.

*Analysis* calculated for $C_{13}H_{13}Cl_2N_3O_3$
Theory: C, 47.30; H, 3.97; N, 12.73;
Found: C, 47.34; H, 3.88; N, 12.60

Example 6

2-(2,6-Dichloro-4-trimethylammoniomethylphenylimino)imidazolidine bromide Hydrobromide

A solution of 2-(4-bromomethyl-2,6-dichlorophenylimino)imidazolidine hydrobromide (2.5 g, 6.2 mmol) in ethanol (100 $cm^3$) was added over 0.8h to stirred ethanolic trimethylamine (33%; 16 $cm^3$). The mixture was stirred at room temperature overnight, concentrated to an oil, which was then stirred with acetonitrile and the insoluble solid filtered. The solid was dissolved in hot methanolic hydrogen bromide and reprecipitated as colourless prisms (2.19 g, 92% yield) by addition of acetone. The product was identified as the title compound, m.p. 270—5°C (dec.).

*Analysis* calculated for $C_{13}H_{20}Br_2Cl_2N_4$
Theory: C, 33.72; H, 4.35; N, 12.10 $Br^-$, 34.51%
Found: C, 33.81; H, 4.38; N, 11.69; $Br^-$, 34.40%

Formulation of the Compounds for Veterinary Administration

*Formulation 1*

Compound of Example 1, Bolus

Boluses of the following composition were prepared:

| | |
|---|---|
| Compound of Example 1 | 100 mg |
| Microcrystalline cellulose | 500 mg |
| Corn starch | 250 mg |
| Magnesium stearate | 25 mg |
| Lactose, anhydrous | to 2500 mg |

The ingredients were passed through a 500 micron mesh stainless steel screen and blended in a suitable blender. The resultant compression mix was compressed directly on a tabletting machine to give tablets each containing 100 mg of the compound of Example 1.

*Formulation 2*

Oral Doser 5 mg/g

1 Kg of the following composition was prepared:

| | % by wt. |
|---|---|
| Compound of Example 1 | 0.5 |
| Aluminium stearate | 6.0 |
| Sunflower oil | to 100 |

The aluminium stearate was dispersed with stirring in a portion of the sunflower oil heated to 115<C. The dispersion was added to the rest of the sunflower oil heated to 140<C. The gel was stirred at 130<C for 15 minutes and then allowed to cool without stirring to room temperature. The milled compound of Example 1 was dispersed in the cooled gel base and then passed through a colloid mill to produce a fine, homogenous dispersion. The dispersion was filled into plastic bottles fitted with a dosing pump.

*Formulation 3*

Injection 5 mg/ml

1 Litre of the following composition was prepared:

| | % w/v |
|---|---|
| Compound of Example 1 | 0.5 |
| Sodium chloride | 0.5 |
| Water for injections | to 100 |

The compound of Example 1 and sodium chloride were dissolved in the water for injections and the solution was filtered and sterilised by membrane filtration. The sterile solution was filled into glass ampoules.

*Formulation 4*

Soluble Powder

1 kg of following composition was prepared:

| | % by wt. |
|---|---|
| Compound of Example 1 | 3.5 |
| Lactose | to 100 |

The compound of Example 1 and lactose were sieved and mixed together in a suitable blender to give a

homogenous powder. The powder was filled into jars. The powder was used at the rate 0.5 g per gallon of drinking water to medicate pigs.

*Formulation 5*

Oral Rehydration Formulation containing the compound of Example 1

1 kg of the following composition was prepared by mixing together the ingredients in dry powder form:

| | |
|---|---|
| Glycine | 10.3 % |
| Dextrose (anhydrous) | 67.6 |
| Sodium Chloride | 14.3 |
| Potassium Dihydrogen Phosphate | 6.8 |
| Citric Acid | 0.8 |
| Tri-potassium Citrate | 0.2 |
| Compound of Example 1 | 0.15 |

60 g of the composition was then dissolved in 2 litres of water and fed to diarrhoeic calves.

*Formulation 6*

The following formulation may be prepared by the method set out below:

| | |
|---|---|
| Compound of Example 1 | 0.5% w/v |
| Bentone 38 (1) | 1.5% w/v (ie 1.5 g/100 ml) |
| Propylene Carbonate | 0.6% w/v |
| Phamasorb (2) | 10% w/v |
| Phosphoric Acid (3) | 0.1% w/v |
| Ampicillin Trihydrate | 6.0% w/v as free acid |
| Soya-Bean Oil | to 100% |

(1) Bentone 38 is an amide derivative of bentonite
(2) Pharmasorb is a brand of activated Attapulgite,
(3) The phosphoric acid is present to balance the alkaline pH of the Bentone.

The Bentone was dispersed in the soya-bean oil, and when thoroughly distributed, the propylene carbonate was added with high speed mixing, followed by colloid milling to produce the base. Into this base was first mixed the phosphoric acid, and then the pharmasorb, the penicillin, and the compound of Example 1 and the resultant suspension was then passed through a colloid mill once more.

*Formulation 7*

| | |
|---|---|
| Compound of example 1 | 6% w/v |
| Citric acid | 75% w/v |
| Sodium citrate* | 19% w/v |

*Sodium citrate may be replaced by sodium acetate or propionate.

The above formulation is added to oral rehydration solutions at the rate of 0.2—0.8 g/l and is fed to diarrhoeic calves.

BIOLOGICAL DATA

Protection of Neonatal Mice from Lethal Enteropathogenic *E Coli* Infection

4 day old mice were orally dosed with 50 µl of phosphate buffered saline containing $1 \times 10^5$ organisms/ml of *E coli* B44 (09:K90:K99) an enteropathogenic strain originally isolated from a scouring calf. The mice were then dosed b.i.d. with either placebo or drug for four days commencing 16 hours after infection. The animals were left with their mothers throughout the experiment and a daily record of deaths was made. The experiment was terminated when no mortality was seen over a 24 hour period. (Usually 7—10 days after infection). The mortality in the drug group was then compared with the mortality in the placebo group using the following formula:

$$\% \text{ Reduction in mortality} = \left[\frac{Mp - Md}{Mp}\right] \times 100$$

where M = mortality in group receiving placebo
Md = mortality in group receiving drug

Statistical analysis was performed using $2 \times 2$ contingency tables (single tailed 'p'). Results are given in Table 1.

TABLE 1

| Treatment | No of mice infected | No of mice dead after 10 days | % Mortality | % Protection by drug |
|---|---|---|---|---|
| Control | 70 | 38 | 54 | — |
| Example I (50 mg/kg) | 68 | 20 | 29 | 46* |
| Example I (10 mg/kg) | 68 | 22 | 32 | 40* |
| Control | 89 | 75 | 84 | — |
| Example 6 (10 mg/Kg) | 73 | 52 | 71 | 15 |

*9 p 0.01 compared to control mortality.

**Claims**

1. A compound of formula (I):

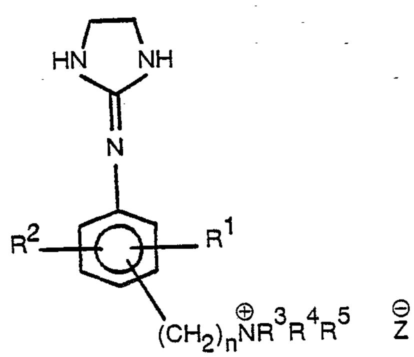

(I)

wherein

$R^1$ and $R^2$ are the same or different and each is selected from halogen, alkyl, haloalkyl, alkoxy and alkoxyalkyl,

$R^3$, $R^4$ and $R^5$ are the same or different and each is alkyl;

wherein the alkyl and alkoxy groups each have from 1 to 6 carbon atoms; those having 3 or more carbon atoms may be straight or branched chains and wherein the haloalkyl groups have 1, 2 or 3 halogen atoms;

n is 0, 1, 2 or 3; and

Z is a pharmaceutically or veterinarily acceptable anion,

and salts thereof.

2. A compound as claimed in claim 1 wherein $R^1$ and $R^2$ are the same and each is methyl or chloro.

3. A compound as claimed in claim 1 wherein $R^1$ and $R^2$ are located in the 2,6 positions in relation to the imidazolidinimino group.

4. A compound as claimed in claim 1 and selected from 2 - (2,6 - Dichloro - 4 - triethylammoniomethylphenylimino)imidazolidine bromide and 2 - (2,6 - Dichloro - 4 - trimethylammoniomethylphenylimino)-imidazolidine bromide and salts thereof.

5. A process for producing a compound of formula (I), as defined in claims 1, which comprises either

(a) reacting a compound of formula (II)

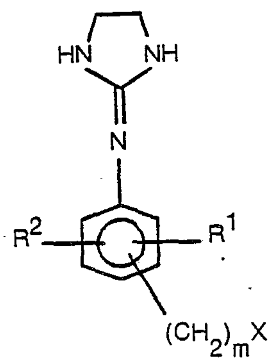

(II)

or a salt thereof,

wherein

$R^1$, and $R^2$ are as defined in relation to formula (I) and m is 1, 2 or 3, and

X is a leaving group with a compound of formula (III)

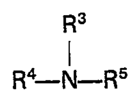

(III)

wherein $R^3$, $R^4$ and $R^5$ are as defined in relation to formula (I) or

(b) reacting a compound of formula (IV)

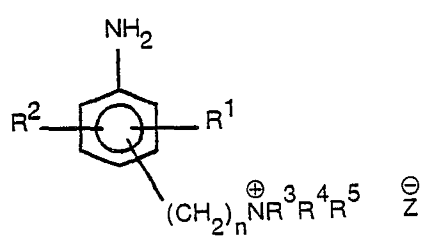

(IV)

wherein $R^1$, $R^2$, $R^3$, $R^4$, $R^5$, n and Z are as defined in relation to formula (I) with a compound of formula (V)

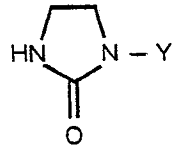

(V)

wherein Y is a protecting group

and thereafter removing the protecting group, or

(c) reacting a compound of formula (VI):

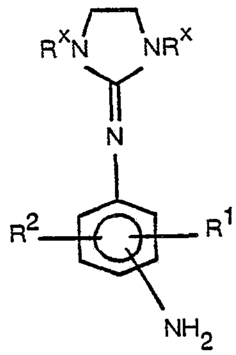

(VI)

wherein $R^1$ and $R^2$ are as defined in relation to formula (I) and $R^x$ is a suitable protecting group with one or more alkyl halides and thereafter removing the protecting groups $R^x$.

6. A process as claimed in claim 5 wherein a compound of formula (II) wherein X is halogen, is reacted with a compound of formula (III) in an organic solvent at ambient temperature.

7. A process as claimed in claim 6 wherein a compound of formula (II) wherein X is bromine, or a hydrohalide salt thereof, is reacted with a compound of formula (III) in a lower alkanol at ambient temperature.

8. A pharmaceutical or veterinary composition comprising a compound of formula (I) as defined in claim 1 and a pharmaceutically or veterinarily acceptable carrier therefor.

9. A formulation for treating diarrhoea which comprises an effective non-toxic amount of a compound of formula (I) as defined in claim 1 and an oral rehydration composition comprising a pharmacologically acceptable aqueous solution containing at least 0.5% w/v of an actively absorbed monosaccharide, at least 25 m*M* sodium ions and having an osmolarity less than 500 m Osmolar.

10. A compound of formula (I) as defined in claim 1 for use in treating the human or animal body.

**Patentansprüche**

1. Eine Verbindung der Formel (I):

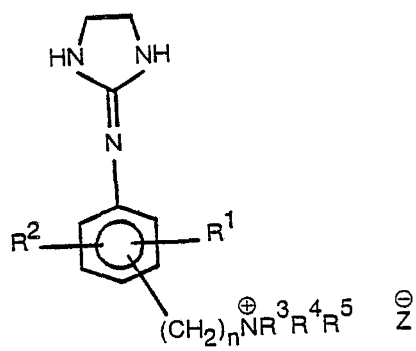

(I)

in welcher $R^1$ und $R^2$ gleich oder verschieden sind und jedes davon ausgewählt ist aus Halogen, Alkyl, Haloalkyl, Alkoxy und Alkoxyalkyl,

$R^3$, $R^4$, und $R^5$ gleich oder verschieden sind und jedes davon ein Alkyl ist,

wobei die Alkyl- und Alkoxygruppen jeweils 1 bis 6 Kohlenstoffatome aufweisen, diejenigen Gruppen, die 3 oder mehr Kohlenstoffatome aufweisen, geradkettig oder verzweight sein können, und wobei die Haloalkylgruppen 1, 2 oder 3 Halogenatome aufweisen können,

n den Wert 0, 1, 2 oder 3 hat, und

Z ein pharmazeutisch oder veterinärmedizinisch verträgliches Anion ist,

und Salze davon.

2. Eine Verbindung wie in Anspruch 1 beansprucht, in welcher $R^1$ und $R^2$ gleich sind und jedes davon eine Methylgruppe oder ein Chloratom darstellt.

3. Eine Verbindung wie in Anspruch 1 beansprucht, in welcher $R^1$ und $R^2$ sinch in der 2- und 6-Stellung in bezug auf die Imidazolidiniminogruppe befinden.

4. Eine Verbingund wie in Anspruch 1 beansprucht und ausgewählt aus 2 - (2,6 - Dichlor - 4 - triäthyl-ammoniomethylphenylimino)-imidazolidinbromid und 2 - (2,6 - Dichlor - 4 - trimethyl - ammoniomethylphenylimino) - imidazolidinbromid und Salzendavon.

5. Ein Verfahren zur Herstellung einer Verbindung der Formel (I), wie in Anspruch 1 definiert, welches Verfahren entweder

a) die Umsetzung einer Verbindung der Formel (II)

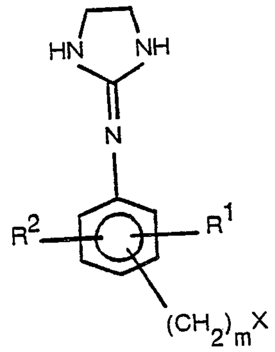

(II)

oder eines Salzes derselben,
in welcher $R^1$ und $R^2$ wie in bezug auf Formel (I) definiert sind und m den Wert 1, 2 oder 3 hat, und X eine abspaltbare Gruppe ist,
mit einer Verbindung der Formel (III)

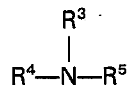

(III)

in welcher $R^3$, $R^4$ und $R^5$ wie in bezug auf Formel (I) definiert sind, oder
b) die Umsetzung einer Verbindung der Formel (IV)

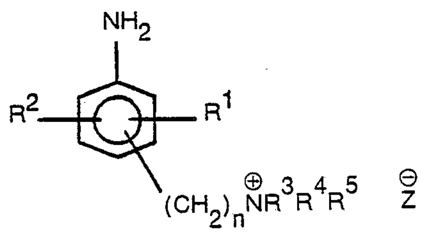

(IV)

in welcher $R^1$, $R^2$, $R^3$, $R^4$, $R^5$, n und Z wie in bezug auf Formel (I) definiert sind,
mit einer Verbindung der Formel (V)

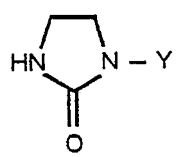

(V)

in welcher Y eine Schutzgruppe ist,
und anschließend die Entfernung der Schutzgruppe, oder
c) die Umsetzung einer Verbindung der Formel (VI):

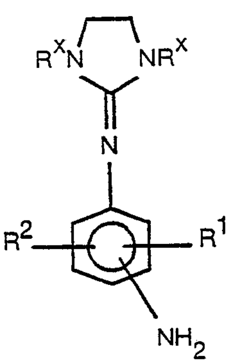

(VI)

in welcher $R^1$ und $R^2$ wie in bezug auf Formel (I) definiert sind und $R^x$ eine geeignete Schutzgruppe ist,
mit einem oder mehreren Alkylhalogenid(en),

und anschließend die Entfernung der Schutzgruppen $R^x$ umfaßt.

6. Ein Verfahren wie in Anspruch 5 beansprucht, in welchem eine Verbindung der Formel (II), in welcher X ein Halogen ist, mit einer Verbindung der Formel (III) in einem organischen Lösungsmittel bei Raumtemperatur umgesetzt wird.

7. Ein Varfahren wie in Anspruch 6 beansprucht, in welchem eine Verbindung der Formel (II), in welcher X Brom oder ein Hydrohalogenidsalz davon ist, mit einer Verbindung der Formel (III) in einem niederen Alkanol bei Raumptemperatur umgesetzt wird.

8. Eine pharmazeutische oder veterinärmedizinische Zusammensetzung umfassend eine Verbindung der Formel (I) wie in Anspruch 1 beansprucht und einen pharmazeutisch oder veterinärmedizinisch verträglichen Träger davon.

9. Eine Rezeptur zur Behandlung von Diarrhöe, welche eine wirksame nicht-toxischen Menge einer Verbindung der Formel (I), wie in Anspruch 1 beansprucht, und eine für die orale Verabreichung geeignete Rehydratisierungszusammensetzung, umfassend eine pharmakoligisch verträgliche wäßrige Lösung enthaltend mindestens 0,5% (Gew./Vol.) eines aktiv absorbierten Monosaccharids, mindestens 25 mM Natriumionen und mit einer Osmolarität von weniger als 500 m Osmolar, enthält.

10. Eine Verbindung der Formel (I) wie in Anspruch 1 beansprucht, zur Verwendung bei der Behandlung des menschlichen oder tierischen Körpers.

**Revendications**

1. Composé de formule (I)

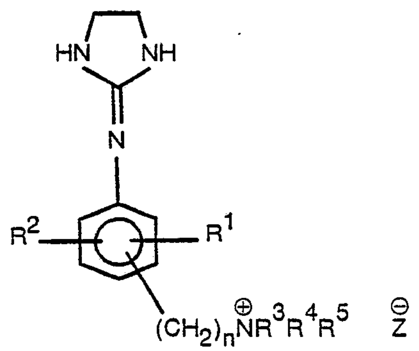

(I)

dans laquelle $R^1$ et $R^2$ sont identiques ou différents et sont choisis chacun parmi un atome d'halogène, un groupe alcoyle, haloalcoyle, alcoxy et alcoxyalcoyle,

$R^3$, $R^4$, et $R^5$ sont identiques ou différents et sont chacun un groupe alcoyle;

où les groupes alcoyle ou alcoxy ont chacun 1 à 6 atomes de carbone; les groupes ayant 3 atomes de carbone ou plus peuvent être à chaîne droite ou ramifiée et les groupes haloalcoyles ont 1, 2 ou 3 atomes d'halogène;

n est 0, 1, 2 ou 3; et

Z est un anion acceptable du point de vue pharmaceutique ou vétérinaire, et ses sels.

2. Composé suivant la revendication 1, caractérisé en ce que $R^1$ et $R^2$ son identiques et sont chacun un groupe méthyle ou un atome de chlore.

3. Composé suivant la revendication 1, caractérisé en ce que $R^1$ et $R^2$ sont situés en positions 2, 6 par rapport au groupe imidozolidinimino.

4. Composé suivant la revendication 1 et choisi parmi le bromure de 2-(2,6-dichloro-4-triéthylammoniométhylphénylimino) imidazolidine et le bromure de 2-(2,6-dichloro-4-triméthylammoniométhylphylimino)-imidazolidine et leurs sels.

5. Procédé de production d'un composé de formule (I) telle qu'elle est définie dans la revendication 1, caractérisé en ce qu'il comprend:

(a) la réaction d'un composé de formule (II)

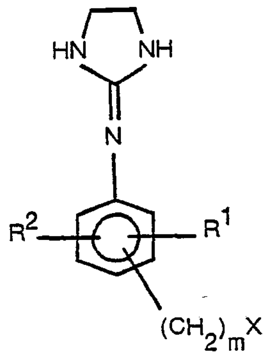

(II)

ou d'un sel de celui-ci, dans laquelle $R^1$ et $R^2$ sont tels que définis à propose de la formule (I) et m est 1, 2 ou 3; et
X est un groupe mobile, avec un composé de formule (III)

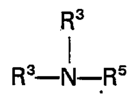

(III)

dans laquelle $R^3$, $R^4$ et $R^5$ son tels que définis à propos de la formule (I), ou bien
(b) la réaction d'un composé de formule (IV)

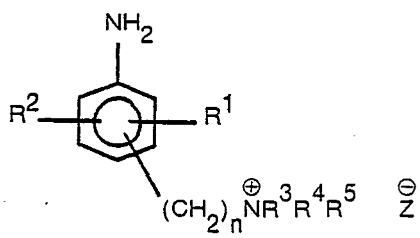

(IV)

dans laquelle $R^1$, $R^2$, $R^3$, $R^4$, $R^5$, n et Z sont tels que définis à propos de la formule (I) avec un composé de formule (V)

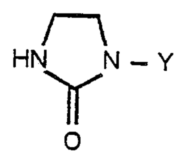

(V)

dans laquelle Y est un groupe protecteur et en suite, l'élimination du groupe protecteur, ou bien
(c) la réaction d'un composé de formule (VI)

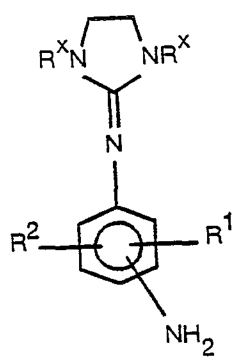

(VI)

dans laquelle $R^1$ et $R^2$ sont tels que définis à propos de la formule (I) et $R^x$ est un grope protecteur convenable, avec un ou plusieurs halogénures d'alcoyles et ensuite, l'élimination des groupes protectures $R^x$.

6. Procédé suivant la revendication 5, caractérisé en ce qu'on fait réagir un composé de formule (II) où

X est un atome d'halogène, avec un composé de formule (III) dans un solvant organique à la température ambiante.

7. Procédé suivant la revendication 6, caractérisé en ce qu'on fait réagair un composé de formule (II) où X est un atome de brome ou un halohydrate de celui-ci, avec un composé de formule (III) dans un alcanol inférieur à la température ambiante.

8. Composition pharmaceutique ou vétérinaire comprenant un composé de formule (I) suivant la revendication 1 et un support pour celui-ci, acceptable du point de vue pharmacetique ou vétérinaire.

9. Formulation pour traiter la diarrhée, qui comprend une quantité efficace non-toxique d'un composé de formule (I) suivant la revendication 1 et une composition de réhydration orale comprenant une solution aqueuse acceptable du point de vue pharmaceutique, contenant au moins 0,5% en poid/volume d'un monosaccharide absorbé activement, au moins 25 mM d'ion sodium et ayant une osmolarité inférieure à 500 m Osmolaire.

10. Composé de formule (I) suivant la revendication 1, utile dans le traitement du corps humain ou animal.